# EUROPEAN PATENT APPLICATION

(11) **EP 4 800 697 A1**
(43) Date of publication of application: **02.09.2026**
(21) Application number: 26160613.1
(22) Date of filing: 25.02.2026
(51) Int. Cl.: G16H 20/17, G16H 40/67, A61M 5/14, A61M 5/142, A61M 5/168, A61M 5/172

(54) **SYSTEMS AND METHODS FOR MAINTAINING HYDRATION LEVELS DURING MULTIPLE INFUSIONS**

(30) Priority: 26.02.2025 US 202563763744 P
(71) Applicant: Fresenius Kabi USA, LLC, Lake Zurich, IL 60047 (US)
(72) Inventor: POWERS, Benjamin, Lake Zurich, 60047 (US)
(74) Representative: Maikowski & Ninnemann Patentanwälte Partnerschaft mbB

(57) **Abstract**

Systems and methods for maintaining hydration levels in a patient undergoing multiple drug infusions are disclosed. A plurality of drug infusion devices are in flow and electronic communication with a designated hydration infusate device for monitoring the drug infusions and for maintaining the level of hydration of the patient.

## Description

### Field of the Disclosure

The present disclosure is directed to systems and methods for administering drugs or other medicaments to a patient. More particularly, the present disclosure is directed to systems and methods for administering multiple drugs or other medicaments to a patient using a plurality of infusion devices. Even more particularly, the present disclosure is directed to systems and methods for maintaining adequate hydration levels in a patient receiving multiple infusions of such drugs or medicaments.

### Background

Infusion pumps are used to administer drugs and other medicaments to patients, typically in a clinical setting. An infusion pump provides a controlled amount of the medicament over time to the patient. The amount and infusion rate of the drug or medicament are administered pursuant to parameters entered by a clinician into the pump using a pump user interface or by other delivery means.

Certain patients, in particular critically ill patients and/or neonates, may be subject to multiple infusions of drugs/medicaments, thereby requiring association of the patient with multiple infusion devices (pumps), each programmed to deliver a specific drug/medicament according to a specific delivery protocol. Such protocols may include selected adjustments in the frequency of drug delivery, the volume of drug delivered and/or the rate of drug delivery. in addition, certain protocols may generate alerts or alarms when the infusion deviates from a pre-programmed parameter or if the patient experiences a negative reaction to the administration of the drug or the infusion overall. Certain protocols may intentionally adjust or alter the rates, volumes and frequency of a given drug infusion.

Where multiple drug infusions are required, an adjustment (expected or unexpected) in the infusion of one drug will alter the total fluid intake by the patient. Insufficient fluids can lead to dehydration (i.e. becoming hypotonic) Conversely too much fluid can lead to overhydration (i.e. becoming hypertonic), which may be particularly problematic in critically ill patients.

Attempting to manually maintain total hydration levels is challenging for the clinician due to both complexity of the setup as well as the periodic and at times unexpected flow rate changes in the one or more drug infusions. Moreover, the dosing of drugs is often programmed and displayed in clinical units (e.g. mcg/kg/min) that are not intuitively translated into volumetric units (i.e. mL/hr) that are used to assess fluid load to the patient. For these reasons it is very difficult for clinicians to maintain consistent fluid delivery levels in order to maintain suitable hydration to a patient undergoing multiple infusions.

Thus, it would be desirable to provide a system and method that monitor each of the multiple drug infusions, determine whether an adequate hydration load is being provided to the patient and, if necessary, adjust the delivery of a hydration infusate to maintain such adequate hydration load. The systems and methods disclosed herein address this need.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a system for delivering multiple drug infusions to a patient is disclosed. The system includes a plurality of infusion devices each configured for flow communication with the vascular system of patient wherein at least one of said infusion devices is designated as a hydration infusate device further configured for delivering a hydration infusate to the patient.

In another aspect, a method for maintaining hydration levels in a patient receiving multiple drug infusions is disclosed. The method includes delivering selected doses of a plurality of drugs/medicaments to a patient from a plurality of drug infusion devices. The method includes establishing electronic communication among the plurality of drug infusion devices and between the plurality of drug infusion devices and one or both of (1) an hydration infusate device configured to deliver a hydration infusate to the patient and (2) a remote server that is in communication with an infusion device configured to deliver a hydration infusate to the patient. The method further includes establishing fluid flow communication between the plurality of drug infusion devices, the hydration infusate device and the patient. In accordance with the method, the hydration infusate delivery device receives infusion information from the plurality of drug infusion devices. Based on such infusion information, the hydration infusate delivery device establishes a target level of total hydration. The method includes delivering a selected volume of the hydration infusate at a selected infusion rate based on the infusion information received from the plurality of drug infusion devices.

### Brief Description of the Drawings

Figure 1 is a schematic diagram of one embodiment of a system for maintaining hydration levels in a patient undergoing multiple drug infusions;
Figure 2 is a schematic diagram of another embodiment of a system for maintaining hydration levels in a patient undergoing multiple drug infusions; and
Figure 3 is a front view of an infusion device suitable for use in the systems and methods described herein.

### Detailed Description of the Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

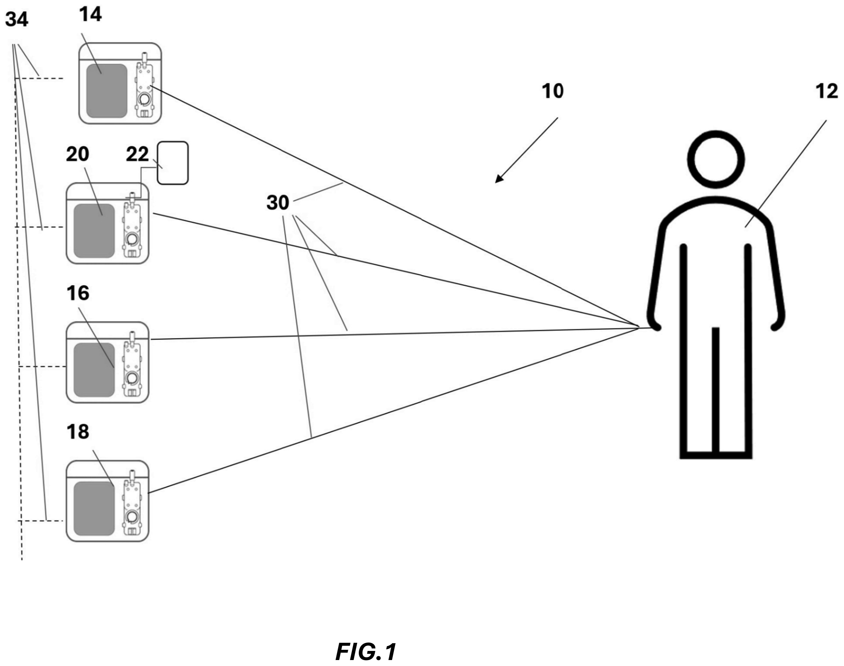

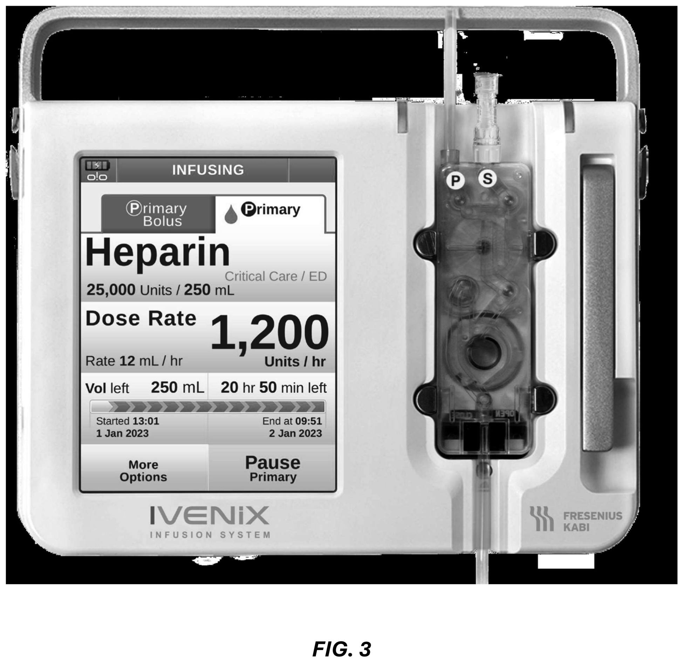

Fig. 1 shows a schematic diagram of system 10 for maintaining hydration levels in a patient 12 undergoing multiple infusions of a drug or medicament. The system includes a plurality of infusion devices (pumps) 14, 16, 18 and 20. Shown in Fig. 1 are four infusion devices (pumps), although more or fewer infusion devices may be used in accordance with the present system and method. One example of a suitable infusion device for use in the systems and methods disclosed herein is the Ivenix Large Volume Pump (LVP) as shown in Fig. 3, and available from Fresenius Kabi USA of Lake Zurich, Illinois. As generally shown in Fig. 3, infusion device includes a housing, a touch/display screen for user interface and a pneumatic pump system. A fluid administration set in the form of a rigid cassette incorporating inlets and an outlet and a diaphragm pump (pneumatically driven) is incorporated into the cassette. The infusion device may include a drug library from which the drugs for administration/infusion may be selected.

As further shown in Fig. 1, the plurality of infusion devices are in fluid flow communication with patient 12 via tubing sets that define flow paths 30 from the infusion device (14, 16, 18 and 20) to the vascular system of patient 12. Vascular access is achieved through a vascular access device such as a needle or cannula with one or more Y-connectors for receiving multiple infusion lines (flow paths) from the plurality of infusion devices.

Infusion devices 14, 16, 18 and 20 may be pre-programmed to deliver a selected dose of a drug/medicament or other fluid to the patient. Programming includes inputting a selected dose, flow rate of a specific drug (selected from an available drug library) or other fluid for administration to a patient, and association of the patient with each of the infusion devices.

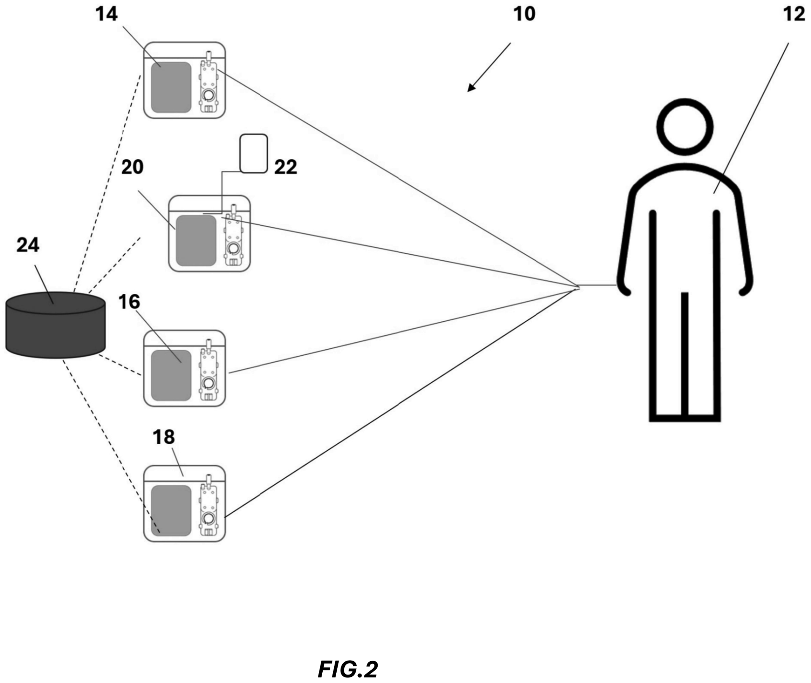

In accordance with the present disclosure, as shown in broken lines 34, all of the infusion devices are in direct electronic communication with each other and/or indirect electronic communication by a remote server 36, as shown in Fig. 2. Communication may be wireless such as by Wi Fi or Bluetooth. In accordance with a further aspect of the present disclosure, multiple infusion devices may be I associated with a patient and associated with one another (by physical proximity and detectability) using RFID, WiFi, Bluetooth, etc, This way the infusion status of the other devices can be communicated to one of the infusion devices (e.g., 20) which can be designated and programmed as a hydration infusate device. The hydration infusate device delivers a non-drug hydration infusate and provides active management and maintenance of the total hydration load to the patient, as described in greater detail below.

Inasmuch as the infusion devices 14, 16, 18 and 20 are able to communicate with one another, hydration infusate device 20 is configured to receive infusion information, such as flow rate, volume etc. from the other drug infusion devices 14, 16, 18. Based on such infusion information such as flow rate and volume of the drug being administered by devices 14, 16, 18, hydration infusate device 20 will determine a total hydration load for the multiple infusions.

Hydration Infusion device 20 may be further configured to continuously or periodically receive infusion information from devices 14, 16 and 18 during the multiple infusions. Hydration infusate device 20 will use and monitor information from such other infusions being delivered to the patient to automatically vary the delivery rate of the hydration fluid or to notify the operator that a deviation from the established hydration load or other parameter such as flow rate has occurred so that the operator can manually adjust the rate of the hydration fluid, as necessary. For example, as new infusions are added, existing infusions completed, or as infusion devices (pumps) transition to a "keep vein open" or KVO condition, or as pumps are restarted (following an alarm condition as discussed below or other interruption in the infusion), hydration infusion device 20 will automatically adjust the output rate of the hydration infusate in order to maintain the constant overall fluid delivery rate to the patient. If the total hydration load is below what is needed to maintain adequate hydration to the patient, hydration infusate device 20 will be activated to deliver a non-drug infusate, such as normal saline, Lactated Ringers solution, or a dextrose solution from container 22, to the patient in an amount and at a rate sufficient to meet the target level of hydration. Alternatively, if the total infusion rate across infusion devices 14, 16, 18 and 20 exceeds the total hydration target (including the rate of the hydration infusate device), the hydration device 20 may lower its delivery rate to a selected threshold (such as "0" or a minimum KVO rate). If after such adjustments, the total infusion rate still exceeds the target, the system would generate an audible and/or visual alarm that the target fluid intake was being exceeded, allowing the clinician to intervene. Where infusion device 20 is designated as the hydration infusate device, the drug library for device 20 may be configured such that only certain fluids may be programmed for use in a hydration management device. For example, a fluid containing medication would not be acceptable for use in automatically varying fluid delivery.

### Other Aspects

Aspect 1. A system for delivering multiple drug infusions to a patient comprising a plurality of infusion devices each configured for flow communication with the vascular system of patient wherein at least one of said infusion devices is designated as a hydration infusate device further configured for delivering a hydration infusate to the patient.

Aspect 2. The system of Aspect 1 wherein said hydration infusate device comprises a controller configured to receive infusion information from one or more of said plurality of infusion devices.

Aspect 3. The system of any one of Aspects 1 and 2 wherein said hydration infusate device controller is configured to continuously receive infusion information from said one or more of said plurality of infusion devices.

Aspect 4. The system of any one of Aspects 1 through 3 wherein hydration infusate device controller is configured to maintain a pre-selected and consistent overall fluid delivery to said patient.

Aspect 5. The system of any one of Aspects 2 through 4 wherein said hydration infusate device is configured to vary the rate of delivery of said infusate based on infusion information received from said one or more of said plurality of infusion devices.

Aspect 6. The system of any one of Aspects 1 through 5 wherein said plurality of infusion devices are configured for delivery of a drug infusate and said hydration infusate to the same patient.

Aspect 7. The system of any one of Aspects 1 through 6 wherein said hydration pump is configured to generate an alarm if a total infusion rate is exceeded.

Aspect 8. The system of any one of Aspects 1 through 7 wherein said designated hydration infusate device is configured such that it cannot deliver a drug or medicament.

Aspect 9. The system of Aspect 8, wherein said hydration infusate comprises one or more of saline, Lactated Ringers solution, or a dextrose solution.

Aspect 10. The system of any one of Aspects 1 through 9 further comprises a remote server in electronic communication with each of said plurality of infusion devices.

Aspect 11. A method for maintaining hydration levels in a patient receiving multiple drug infusions comprising: a. delivering selected doses of a plurality of drugs to a patient from a plurality of drug infusion devices; b. establishing electronic communication among said plurality of drug infusion devices and between said plurality of drug infusion devices and one or both of (1) a hydration infusate device configured to deliver a hydration infusate to said patient and (2) a remote server that is in communication with an infusion device configured to deliver a hydration infusate to said patient; c. establishing fluid flow communication between said plurality of drug infusion devices, said hydration infusate device and said patient; d. receiving infusion information from said plurality of drug infusion devices at said hydration infusate infusion device; e. establishing a target level of total hydration based at least in part on infusion information received from said drug infusion devices; and f. delivering a selected volume of said hydration infusate at a selected infusion rate based on infusion information received from said plurality of drug infusion device.

Aspect 12. The method of Aspect 11 comprising adjusting said selected volume and selected infusion rate of said hydration infusate in response to infusion information received by said hydration infusate device from one or more of said plurality of drug infusion device.

Aspect 13. The method of any one of Aspects 11 and 12 wherein said infusion information from said drug infusion devices is wirelessly communicated to said hydration infusate device.

Aspect 14. The method of any one of Aspects 11-13 wherein said infusion information comprises rate of drug infusion, completion of drug infusion, interruption of drug infusion, addition of a new drug infusion, air in the infusion line or other unexpected condition.

Aspect 15. The method of any one of Aspects 11-14 wherein said hydration infusate device generates an alert or alarm when the total infusion rate or volume is above or below the target level of total hydration.

Aspect 16. The method of any one of Aspects 11-15 wherein said hydration infusate comprises one or more of saline, Lactated Ringers solution, or a dextrose solution.

Aspect 17. The method of any one of Aspects 11-16 comprising associating each of said drug infusion devices and said hydration infusion device with a patient.

Aspect 18. The method of any one of Aspects 11-17 wherein infusion information from said plurality of drug infusion devices is continuously provided to said hydration infusion device.

Aspect 19. The method of any one of Aspects 11-18 comprising displaying infusion information on a display screen of said hydration infusate device.

Aspect 20. The method of any one of Aspects 11-19 comprising disabling said hydration infusate device for drug infusion.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A system for delivering multiple drug infusions to a patient comprising a plurality of infusion devices each configured for flow communication with the vascular system of patient wherein at least one of said infusion devices is designated as a hydration infusate device further configured for delivering a hydration infusate to the patient.

2. The system of Claim 1 wherein said hydration pump comprises a controller configured to receive infusion information from one or more of said plurality of infusion devices.

3. The system of any one of Claim 1 and 2 wherein said hydration pump controller is configured to continuously receive infusion information from said one or more of said plurality of infusion devices and/or wherein the hydration infusate device controller is configured to maintain a pre-selected and consistent overall fluid delivery to said patient.

4. The system of any one of Claims 2 and 3 wherein said hydration infusate device is configured to vary the rate of delivery of said infusate based on infusion information received from said one or more of said plurality of infusion devices.

5. The system of any one of Claims 1 through 4 wherein said plurality of infusion devices are configured for delivery of a drug infusate and said hydration infusate to the same patient.

6. The system of any one of Claims 1 through 5 wherein said hydration infusate device is configured to generate an alarm if a total infusion rate is exceeded and/or wherein said designated hydration infusate device is configured such that it cannot deliver a drug or medicament, in particular wherein said hydration infusate comprises one or more of saline, Lactated Ringers solution, or a dextrose solution.

7. The system of any one of claims 1 through 6 further comprises a remote server in electronic communication with each of said plurality of infusion devices.

8. A method for maintaining hydration levels in a patient receiving multiple drug infusions comprising:
a. delivering selected doses of a plurality of drugs to a patient from a plurality of drug infusion devices;
b. establishing electronic communication among said plurality of drug infusion devices and between said plurality of drug infusion devices and one or both of (1) an infusion device configured to deliver a hydration infusate to said patient and (2) a remote server that is in communication with an infusion device configured to deliver a hydration infusate to said patient;
c. establishing fluid flow communication between said plurality of drug infusion devices, said hydration infusate infusion device and said patient;
d. receiving infusion information from said plurality of drug infusion devices at said hydration infusate infusion device;
e. establishing a target level of total hydration based at least in part on infusion information received from said drug infusion devices; and
f. delivering a selected volume of said hydration infusate at a selected infusion rate based on infusion information received from said plurality of drug infusion device.

9. The method of Claim 8 comprising adjusting said selected volume and selected infusion rate of said hydration infusate in response to infusion information received by said hydration infusate infusion device from one or more of said plurality of drug infusion device.

10. The method of any one of Claims 8 and 9 wherein said infusion information from said drug infusion devices is wirelessly communicated to said hydration infusate device and/or wherein said infusion information comprises rate of drug infusion, completion of drug infusion, interruption of drug infusion, addition of a new drug infusion, air in the infusion line or other unexpected condition.

11. The method of any one of Claims 8-10 wherein said hydration infusate device generates an alert or alarm when the total infusion rate or volume is above or below the target level of total hydration.

12. The method of any one of Claims 8-11 wherein said hydration infusate comprises one or more of saline, Lactated Ringers solution, or a dextrose solution.

13. The method of any one of Claims 8-12 comprising associating each of said drug infusion devices and said hydration infusion device with a patient.

14. The method of any one of Claims 8-13 wherein infusion information from said plurality of drug infusion devices is continuously provided to said hydration infusion device and/or wherein the method comprises displaying infusion information on a display screen of said hydration infusate device.

15. The method of any one of Claims 8-14 comprising disabling said hydration infusate device for drug infusion.
